# EUROPEAN PATENT APPLICATION

(11) **EP 3 254 723 A1**
(43) Date of publication of application: **13.12.2017**
(21) Application number: 16174037.8
(22) Date of filing: 10.06.2016
(51) Int. Cl.: A61M 25/00, A61B 17/00, A61M 5/19, B01F 5/00, B05C 17/00

(54) **CATHETER FOR DELIVERING A THERAPEUTIC AGENT INTO A SUBSTRATE**

(71) Applicant: Celyad S.A., 1435 Mont-Saint-Guibert (BE)
(72) Inventor: ALEKSIEJUS, Darius, 1435 Mont-Saint-Guibert (BE); THIBAUT, kyun, 1435 Mont-Saint-Guibert (BE)
(74) Representative: Crease, Devanand John

(57) **Abstract**

An apparatus is provided that is suitable for use as a delivery catheter (12), the apparatus comprising: (a) an elongate shaft (16) that comprises a proximal end and a distal end, wherein the elongate shaft defines at least one lumen; and (b) at least one combining chamber (40) that is contained within the lumen proximate to the distal end of the elongate shaft, the combining chamber having at least a first and a second inlet (26, 28) to allow a first and a second fluid component (2, 4) to enter the combining chamber; and at least one outlet (22) to allow the combined first and second fluid components to exit the combining chamber.

## Description

### FIELD

The invention relates to the field of delivery catheters. More specifically, the invention relates to an injection catheter for delivering a therapeutic agent in a gel form into a substrate and a process for injecting a therapeutic agent in a gel form into a substrate.

### BACKGROUND OF THE INVENTION

Cardiovascular disease is a leading cause of morbidity and mortality worldwide and in contrast to tissues with high reparative capacity, heart tissue is vulnerable to damage that is irreparable by the normal mechanisms of the body. The prevention and treatment of these diseases are thus a major issue and numerous clinical efforts are being made to improve the care and treatment of cardiac disorders.

Regenerative medicine is one current research method for reducing dysfunction of organs, such as the heart, for example *(*Sherman, Cellular Therapy for Chronic Myocardial Disease: Nonsurgical approaches, Basic Appl. Myol. 13(1) 11-14*).* This treatment involves the injection of therapeutic agents directly into the organ through devices such as injection catheters. Heldman et al., Cell Therapy for myocardial infarction: Special delivery, Journal of Molecular and Cellular Cardiology, 2008, 44, 473-476, describes several such delivery devices for use in the heart along with their disadvantages listed according to type of injection (epicardiac, endocardiac, intracoronary or intravenous).

Retention of the therapeutic agent after administration is of the utmost importance. Typically, after injection of a therapeutic agent, a proportion of the injectate is often lost from the site of delivery to surrounding tissue or circulating fluid. This can make it very difficult to predict and then administer an accurate dose to the patient. The loss of therapeutic agent to other areas of the body is also a potential source of undesirable side effects. If the therapeutic agent is expensive or difficult to obtain, for example, modified stem cells for use in regenerative cell therapy, then loss of therapeutic agent is also costly and wasteful.

Retention of therapeutic agent is particularly difficult when injecting into a living muscle, such as the myocardium, that may contract during and after administration. Each muscle contraction effectively squeezes the injected therapeutic agent from the tissue leading to a more pronounced loss of administered therapeutic agent from the site of delivery.

Loss of therapeutic agent from a delivery site may be mitigated through co-administration of a therapeutic agent within a retention medium. A retention medium is generally a viscous bio-erodable gel that forms a matrix within which the therapeutic agent is retained. Two-part retention media that comprise two fluid, flowable, precursors that when mixed undergo a chemical reaction to form a viscous retention medium are known; for example, a hydrogel based on hyaluronan derivatives is commercially available from Contipro a.s.

US 8715265 describes a catheter system for the injection of a therapeutic agent in a two component bio-erodable gel to aid retention of the therapeutic agent at a treatment site. In US 8715265 the bio-erodable gel is either pre-formed and introduced into the catheter by suctioning, or the two part gel is formed by mixing the components as they exit, i.e. exterior to, the catheter. US 8715265 fails to disclose of mixing the two-part gel at the distal end of the catheter immediately prior to injection.

The high viscosity of the pre-formed retention medium prevents it from being passed through the narrow lumens of a typical catheter without risk of blockage. While it is possible to select retention media that are less viscous, the use of low viscosity retention media may detrimentally affect its ability to retain therapeutic agent.

Mixing of the retention media outside of the catheter, e.g. within a substrate, requires that the fluid retention media precursors are first expelled simultaneously from the catheter. This uncontrolled release of liquid components means that the formation of the viscous combined reaction media is prone to exterior influences such as dilution effects and "wash-out" of both the retention media precursors and the therapeutic agent prior to formation of a gel matrix. The risk of dilution of the materials after deposition at the injection site may lead to improper mixing of the components resulting in poor retention media formation. The piercing of tissue with two piercing members would also lead to significant trauma.

Therefore, there remains a need in the art for a catheter apparatus capable of facilitating delivery of a therapeutic agent, such as cells, with the goal of improved retention. The cells administered by the apparatus would be less prone to loss from the treatment site as a result of the mechanical effects induced by the tissue, for example the beating of the heart.

These and other uses, features and advantages of the invention should be apparent to those skilled in the art from the teachings provided herein.

### SUMMARY OF THE INVENTION

In its primary aspect the invention provides an apparatus, suitable for use as a delivery catheter, the apparatus comprising:
(a) an elongate shaft that comprises a proximal end and a distal end, wherein the elongate shaft defines at least one lumen; and
(b) at least one combining chamber that is contained within the lumen proximate to the distal end of the elongate shaft, the combining chamber having at least a first and a second inlet to allow a first and a second fluid component to enter the combining chamber; and at least one outlet to allow the combined first and second fluid components to exit the combining chamber.

Suitably, the apparatus further comprises at least a first and a second lumen that extend to the proximal end of the elongate shaft from the combining chamber, wherein the first and second inlets are in respective fluid communication with the first and second lumens.

Typically, the first and second lumens are connected to a first and a second component reservoir located at the proximal end. Suitably, the first and second component reservoirs comprise a first and a second syringe. Typically, the means of attachment of the first and the second syringe to the respective first and second lumen is via a luer lock coupling.

In an embodiment of the apparatus, in use the first and second fluid components are combined within the combining chamber. Suitably, the first and second fluid components are at least partially mixed within the combining chamber. The first and second fluid components may suitably form a biocompatible polymer when combined. Typically, the first and second fluid components are mixed within the combining chamber to the extent that a polymerisation reaction is initiated in substantially all of the volume of the mixed fluid components. In an embodiment of the apparatus, the biocompatible polymer is a bioerodable gel.

The combined first and second fluid components are suitably dispensed from the apparatus as a fluid. Typically, the flow rate of the first and second fluid components through the apparatus is controlled such that the combined first and second fluid components are dispensed as a fluid. The means of control of the flow rate may be selected from the group consisting of: rate of introduction of the first and second fluid components into the combining chamber; size of the at least two inlets to the combining chamber; size of the at least one outlet from the combining chamber; viscosity of the first and second fluid components; viscosity of mixed first and second fluid components; or a combination thereof.

In an embodiment of the first aspect of the invention, one or more of the first and/or second fluid component contains a therapeutic agent. Suitably, the therapeutic agent is contained in the first fluid component. Alternatively, the therapeutic agent is contained in more than one fluid component. Typically, the combined first and second fluid components that are dispensed from the apparatus contain a therapeutic agent. The therapeutic agent may suitably comprise cells, the cells may be stem cells.

In an embodiment of the apparatus of the first aspect of the invention, the combining chamber defines a dead volume of at least 1µl, optionally at least 5µl. Suitably, the combining chamber defines a dead volume of at most 50µl, optionally at least 15µl. In an embodiment, the combining chamber defines a dead volume in the range of 1µl to 50µl, optionally, in the range of 5µl to 15µl.

In an alternative embodiment, the apparatus further comprises a delivery conduit wherein the delivery conduit receives combined first and second fluid components from the combining chamber via the at least one outlet and directs the combined first and second fluid components to a delivery site.

The delivery site may suitably be within a body tissue, joints, a body cavity or vessel. Optionally, the delivery site is within the myocardium.

In an embodiment of the invention, the delivery conduit moves between a retracted position wherein the distal end of the delivery conduit is located within the central lumen of the apparatus, and an extended position wherein the distal end of the delivery conduit extends outside of the central lumen of the apparatus. The delivery conduit may suitably be connected to the combining chamber and/or extend into the combining chamber. Optionally, the delivery conduit extends from the distal end of the apparatus through the combining chamber to the proximal end of the apparatus.

In an embodiment of the apparatus of the first aspect of the invention, the delivery conduit receives combined first and second fluid components via at least one aperture in the side wall of the delivery conduit. Suitably, the at least one aperture permits fluid communication between the combining chamber and the delivery conduit when the delivery conduit is in the extended position. In addition, or as an alternative, the at least one aperture does not permit fluid communication between the combining chamber and the delivery conduit when the delivery conduit is in the retracted position.

In an embodiment, the delivery conduit comprises a restriction proximal to the aperture in the delivery conduit thereby promoting unidirectional flow of the mixed first and second fluid components through the delivery conduit in a proximal to distal direction. Suitably, the delivery conduit is a hollow needle which in embodiments may be formed of shape-memory metal.

In its secondary aspect the invention provides an elongate shaft assembly for use as a delivery catheter, the elongate shaft assembly comprising:
(a) an elongate body that comprises a proximal end and a distal end, wherein the elongate body comprises at least a first lumen and a second lumen;
(b) at least one mixing chamber that is located at the distal end of the elongate body; and
(c) a delivery conduit connected to the mixing chamber;
wherein the mixing assembly is located in the vicinity of the distal end of the elongate shaft assembly; and wherein the mixing chamber is arranged to be in fluid communication with each of the first and second lumens.

In an embodiment of the second aspect of the invention, the first and second lumens extend through the elongate body to the proximal end of the elongate shaft assembly. Suitably, the first lumen and the second lumen are attached to a respective first fluid component reservoir and a second fluid component reservoir located at the proximal end of the elongate shaft assembly. Typically, the first and second fluid component reservoirs comprise a respective first syringe and a second syringe. Optionally, the means of attachment of the first and the second syringe to the respective first and second lumen is via a luer lock coupling.

In use, in an embodiment, a first fluid component and a second fluid component are at least partially mixed within the mixing chamber to provide mixed first and second fluid components. Suitably, the first and second fluid components form a biocompatible polymer when mixed. Typically, the first and second fluid components are mixed within the mixing chamber to the extent that a polymerisation reaction is initiated in substantially all of the volume of the mixed fluid components. Optionally, the biocompatible polymer is a bioerodable gel.

In an embodiment of the invention, the mixed first and second fluid components are dispensed from the delivery conduit as a fluid. Suitably, the flow rate of the first and second fluid components through the apparatus is controlled such that the mixed first and second fluid components are dispensed as a fluid. Typically, the means of control of the flow rate is selected from the group consisting of: rate of introduction of the first and second fluid components into the at least one mixing chamber; size of the inlets to the at least one mixing chamber; size of the outlet from the at least one mixing chamber; viscosity of first and second fluid components; viscosity of the mixed first and second fluid components; or a combination thereof.

In an embodiment of the second aspect of the invention, one or more of the first and second fluid components contains a therapeutic agent. Suitably, the therapeutic agent is contained in the first fluid component. Alternatively, the therapeutic agent is contained in both the first fluid component and the second fluid component. Optionally, the combined first and second fluid components that are dispensed from the apparatus contain a therapeutic agent. In an embodiment, the therapeutic agent is cells, suitably the cells are stem cells.

In an embodiment, the mixing chamber defines a dead volume of at least 1µl, optionally at least 5µl. Suitably, the mixing chamber defines a dead volume of at most 50µl, optionally at least 15µl.

Alternatively, the mixing chamber defines a dead volume in the range of 1µl to 50µl, optionally, in the range of 5µl to 15µl.

In embodiments, the delivery conduit is an opening in the mixing chamber. Alternatively, the delivery conduit is an elongate hollow member selected from the group consisting of: a lumen, a tube, a hollow needle, a channel, a pipe, a cylinder, a hose, a duct or a spout. Suitably, the delivery conduit moves between a retracted position wherein the distal end of the delivery conduit is located within the elongate shaft assembly of the apparatus, and an extended position wherein the distal end of the delivery conduit extends outside of the elongate shaft assembly of the apparatus. Typically, the delivery conduit is connected to the mixing chamber. Optionally, the delivery conduit extends into the mixing chamber. In embodiments, the delivery conduit extends from the distal end of the apparatus through the combining chamber to the proximal end of the apparatus.

In an embodiment, fluid communication between the mixing chamber and the delivery conduit is via at least one aperture in the side wall of the delivery conduit. Suitably, the at least one aperture permits fluid communication between the mixing chamber and the delivery conduit when the delivery conduit is in the extended position. In addition, or as an alternative, the at least one aperture does not permit fluid communication between the mixing chamber and the delivery conduit when the delivery conduit is in the retracted position.

In an embodiment of the second aspect of the invention, the delivery conduit has a restriction proximal to the aperture in the delivery conduit thereby promoting unidirectional flow of the mixed elongate shaft assembly fluid components through the delivery conduit in a proximal to distal direction. Suitably, the delivery conduit is a hollow needle, optionally, the hollow needle is formed of shape-memory metal.

In embodiments of the first and second aspect of the invention, the apparatus and elongate shaft assembly further comprise a pull-wire that is slidably located within a pull-wire lumen within the elongate shaft. Suitably, the pull-wire extends from the proximal end of the apparatus to be fixedly attached at the distal end of the apparatus. Typically, the pull-wire acts to deflect the distal tip of the apparatus.

In a third aspect the invention provides a percutaneous catheter comprising an apparatus or elongate shaft assembly of the first and second aspect of the invention. Suitably, the percutaneous catheter is an injection catheter.

In a fourth aspect the invention provided a method for directing therapy within the body of a subject comprising using an apparatus or elongate shaft assembly of the first, second or third aspect of the invention. Suitably, the therapy comprises delivery of a pharmaceutical composition. Alternatively, the therapy comprises delivery of a cellular preparation.

### BRIEF DESCRIPTION OF THE DRAWINGS

The invention is further illustrated by the accompanying figures in which:
**Figure 1** is a schematic representation of the mixing or combining chamber of a delivery catheter of an embodiment of the invention;
**Figure 2** is a side sectional view of the distal end portion of a delivery catheter of an embodiment of the invention. The longitudinal axis of the catheter is shown with distal to the left and proximal to the right of the figure;
**Figure 3** is a cross-sectional view of the elongate body of the embodiment of the invention shown in Figure 2
**Figure 4** is an oblique perspective view of the mixing assembly body of an embodiment of the invention.
**Figure 5** shows a number of cross-sectional views of embodiments of the mixing assembly body with varying designs of mixing chamber a pull-wire extension lumen.
**Figure 6** shows a side sectional view of the distal end portion of the delivery catheter of an embodiment of the invention with the delivery conduit in (a) an extended position within the lumen of the catheter, and (b) a retracted position with the delivery conduit projecting from the lumen of the catheter.
**Figures 7a** and **7b** show respectively oblique perspective views of the proximal end wall of the mixing assembly and the distal end wall of the mixing assembly of an embodiment of the invention.

### DETAILED DESCRIPTION OF THE INVENTION

All references cited herein are incorporated by reference in their entirety. Unless otherwise defined, all technical and scientific terms used herein have the same meaning as commonly understood by one of ordinary skill in the art to which this invention belongs.

Prior to setting forth the invention, a number of definitions are provided that will assist in the understanding of the invention.

As used herein, the term "comprising" means any of the recited elements are necessarily included and other elements may optionally be included as well. "Consisting essentially of" means any recited elements are necessarily included, elements that would materially affect the basic and novel characteristics of the listed elements are excluded, and other elements may optionally be included. "Consisting of" means that all elements other than those listed are excluded. Embodiments defined by each of these terms are within the scope of this invention.

As used herein, the terms "distal" and "proximal" are used to refer to orientation along the longitudinal axis of the apparatus. Since the devices of the invention are elongate in nature and conform to a single dimension, in use the distal direction refers to the end of the device furthest away from the operator and the proximal direction the end of the device closest to the operator. It should be noted that the term proximal should not be confused with the term 'proximate', which adopts its conventional meaning of 'near to'.

As used herein, the term "lumen" refers to the opening or the inside space of a hollow tubular element. In the present context, a lumen may define a chamber and may also facilitate the insertion of a second element, or the injection of a fluid, in a duct, a body cavity or vessel.

As used herein, the term "delivery conduit" refers to an outlet through which mixed or combined fluid components may be expelled from the device to a delivery site. The delivery conduit may be an opening, a lumen, a tube, a channel, a pipe, a cylinder, a hose, a duct, or a spout. The delivery conduit may be in the form of a moveable element, for example a hollow needle with a sharpened tip, or a sleeve.

The term "biocompatible" means exhibition of essentially no cytotoxicity or immunogenicity while in contact with body fluids or tissues. As used herein, the term "polymer" refers to oligomers, co-oligomers, polymers and co-polymers, e.g., random block, multiblock, star, grafted, gradient copolymers and combination thereof.

As used herein, the term "gel" means a semi-solid colloidal network or polymer network that is expanded throughout its whole volume by a fluid. A gel does not readily flow or may not flow at all without the influence of a positive pressure or negative pressure. A gel may contain: a covalent polymer network, for example, a network formed by crosslinking polymer chains or by non-linear polymerization; a polymer network formed through the physical aggregation of polymer chains, for example, via hydrogen bonds, crystallization, helix formation, complexation; a polymer network formed through glassy junction points, for example, one based on block copolymers; lamellar structures including mesophases for example, soap gels, phospholipids and clays; particulate disordered structures, for example, a flocculent precipitate usually consisting of particles with large geometrical anisotropy, such as in V₂O₅ gels and globular or fibrillar protein gels.

As used herein, the term "gel-forming components" means components that are not in themselves a gel, but when mixed, will form a gel.

In its broadest configuration the invention provides for apparatus in the form of medical devices each comprising an elongate shaft assembly, typically in the form of a catheter that comprises functional elements at the distal portion and a user or operator interface at the proximal terminus. The user interface may comprise a handle, handle assembly or hub.

The elongate shaft assembly is suitably configured for percutaneous use, such as via the intravascular, intra-venous and intra-arterial modes; that involves introduction into a hollow anatomical vessel within the body of a subject animal or patient. The handle assembly remains outside, i.e. external to, the body of the subject. In a specific embodiment of the invention the elongate shaft comprises or consists of a catheter, suitably comprising a tube portion that may define one or more lumens located coaxially within the shaft. The catheter may be adapted for use with an associated guide wire in convention over-the-wire (OTW) or monorail configurations. In embodiments where the catheter is adapted for use with a guide wire, the catheter may further comprise an additional lumen that is adapted to accommodate a guide wire. Any such guide wire may be pre-located within the subject in order to facilitate placement of the device when in use. Alternatively, the guide wire may pass through an existing lumen and be removed and replaced with another member prior to operational use.

The device of the present invention is suitable for intravascular use, in particular intracoronary use. In other embodiments of the invention the device may be used within the blood vessels of the abdomen, the head and neck or limbs, or within the ducts of the gastrointestinal or genito-urinary tracts.

In an exemplary embodiment, the present invention relates to an injection catheter that facilitates delivery of a therapeutic agent within a retention medium to a treatment site within the body of an individual. Suitably, the medium enhances the retention of the therapeutic agent at the treatment site. The treatment site may be the myocardium. Retention of therapeutic agent at the treatment site generally increases the efficacy of the therapy.

One or more therapeutic agents may be combined with one or more of the separate fluid components of the retention media. The therapeutic agent is then mixed and held within the matrix of the viscous retention media formed at the delivery site. The injection of therapeutic agent in a viscous retention media provides advantages not only in terms of retention of the therapeutic agent at the delivery site, but may also allow for controlled release of the therapeutic agent from the delivery site and, in the case of where the therapeutic agent includes stem cells, it may improve viability.

The present invention provides a means by which the two or more components of a two-part retention mixture may be combined near the delivery terminus of a catheter so that the mixed components may be expelled in a fluid state before continuing to form a viscous matrix outside of the catheter at the delivery site.

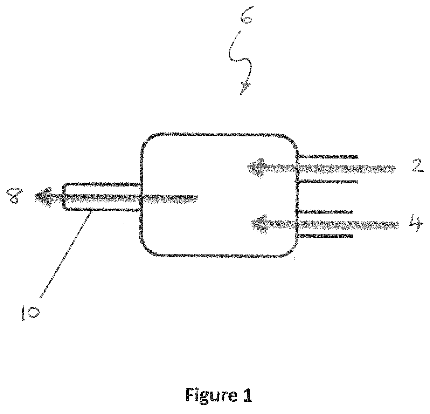

The general principle of one embodiment of the present invention is shown in **Figure 1****.** The separate first and second fluid components 2, 4 enter a mixing chamber 6 through inlets before physically interacting, and at least partially combining or mixing in the chamber 6. The combined first and second fluid components 8 are then expelled through an outlet to the delivery terminus 10 and ultimately the delivery site. One or more therapeutic agents may be present in either or both of the separate first and second fluid components.

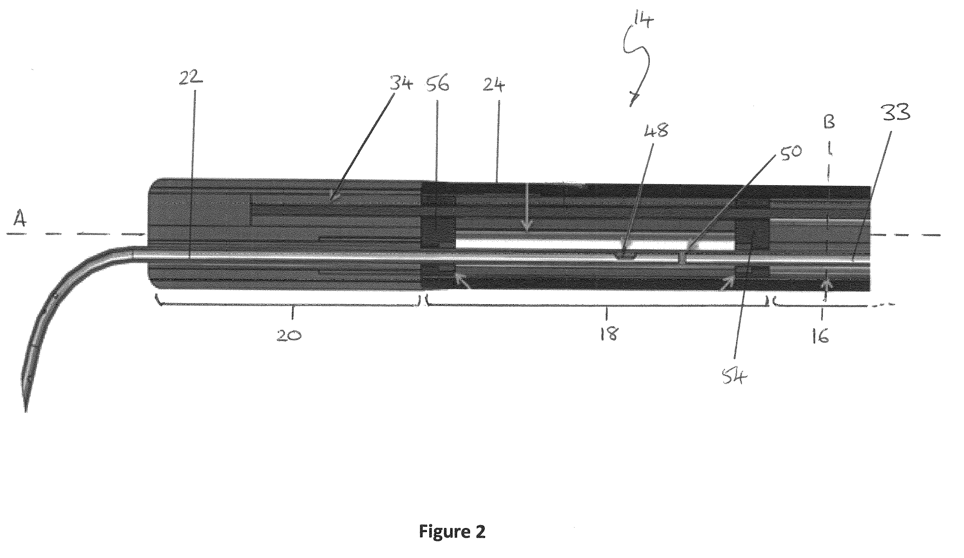

**Figure 2** shows a section view of the distal end of an embodiment of the delivery catheter 12 of the present invention. The delivery catheter of this embodiment comprises an elongate shaft assembly 14 which comprises an elongate body 16, a mixing assembly 18, a distal tip portion 20, and a delivery conduit 22.

A continuous sheath 24 surrounds the elongate body and the mixing assembly 18. The sheath joins to the distal tip portion 20 to provide a uniform, smooth exterior to the delivery catheter 12. In alternative embodiments (not shown) the sheath extends from the proximal end to the distal end of the elongate shaft. All or a portion of the sheath 24 comprise a low friction or lubricious coating that may include, for example, include a fluoropolymer such as a PTFE or parylene.

The elongate body 16 extends along substantially all of the elongate shaft assembly 14 from the proximal end of the delivery catheter 12 to the mixing assembly 16 located at, in the vicinity of, proximate to, near, or close to, the distal end of the delivery catheter 12. As shown in Figure 1, the mixing assembly 6 may be separated from the distal end of the delivery catheter 12 by the distal tip portion 20. In alternative embodiments, the mixing assembly 6may extend to the end of the delivery catheter 12, in which case no distal tip portion is provided.

The elongate shaft assembly 14 of embodiments of the invention may be suitably constructed as catheters in a variety of sizes typically ranging from about 0.15 mm up to about 4 mm in diameter (corresponds to French sizes 0.5 to 12). Suitably the elongate shaft assembly 14 is 2.7mm in diameter (8Fr) to provide for minimal trauma to the tissues and blood vessels of the patient. The length of the elongate shaft assembly 14 is variable, with a typical length of approximately 1m to 1.5m. The elongate body 16 is suitably constructed from a polymeric material such as a silicone rubber or a polymer including thermoplastic elastomer, PEEK, polyimide, high density polyethylene (HDPE), Pebax, and/or nylon; or composites thereof.

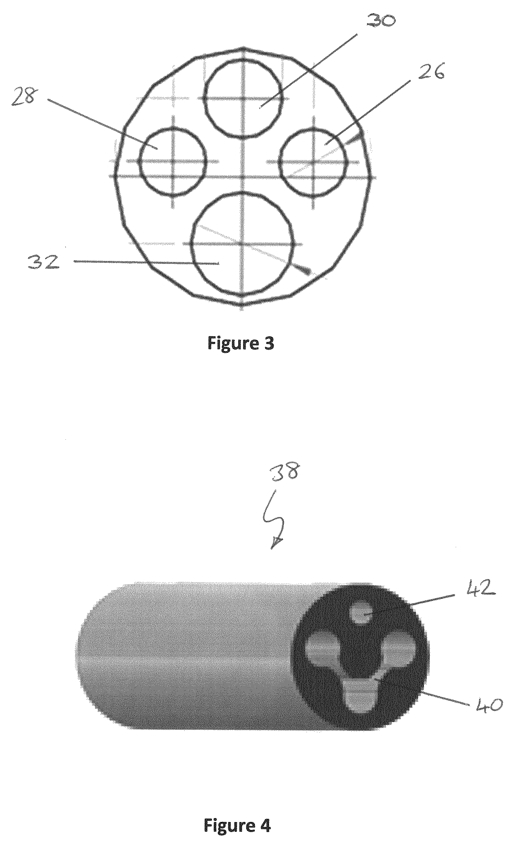

The elongate body 16 comprises one or more chambers located co-axially within the body 16. The chambers may be defined by lumens. Suitably, the elongate body 16 comprises more than one lumen. More suitably the elongate body 16 comprises a plurality of lumens. Most suitably the elongate body 16 comprises between two and four lumens. The lumens within the elongate body 16 are defined according to function and may selected from the group consisting of: fluid component lumens 26, 28, intended for the passage of fluid components along the elongate body; a pull-wire lumen 30, suitable for housing a pull-wire 34; a delivery conduit extension lumen 32, suitable for housing a delivery conduit extension 33; and in some embodiments, a guide-wire lumen to accommodate a guide-wire to locate the delivery catheter 12 within the body prior to use. The arrangement of the lumens in an embodiment of the invention is shown in Figure 3 as a section view of the elongate body of the embodiment of the invention shown in Figure 2 along the line BB.

In the embodiment of the invention shown in Figure 3, the elongate body 16 comprises first and second fluid component lumens 26, 28. The first and second fluid component lumens 26, 28 extend along at least substantially the entire length of the elongate body 16. The first and second fluid component lumens 26, 28 extend from, and thereby provide fluid communication between, the proximal end of the elongate body 16 and the mixing assembly 6at or proximate to the distal end of the elongate shaft assembly 14. The proximal ends of the first and second fluid component lumens (not shown) may engage with separate first and second component reservoirs. Examples of such reservoirs include syringes attached to the first and second fluid component lumens via luer lock attachments. Separate fluid components 2, 4 may pass under pressure or by the force of gravity from the separate first and second reservoirs to the mixing assembly via the first and second fluid component lumens 26, 28. In alternative embodiments of the invention, the elongate body 16 may comprise more than two component lumens meaning that more than two initially separated components can be introduced into the mixing assembly at once.

In an embodiment of the invention, a therapeutic agent may be present in one or more of the fluid components 2, 4. The therapeutic agent may comprise any agent that improves or maintains health, for example, a medicinal compound, small molecule, antibody, other biological or cells. The fluid component in which the therapeutic agent is provided is suitably compatible with the therapeutic agent, i.e. does not significantly or materially reduce or eliminate the therapeutic effect of the agent. More than one therapeutic agent may be provided in separate fluid components, or within a single fluid component. The therapeutic agent may be provided with additional excipients that improve its therapeutic effect or stabilise the agent in the fluid component.

In the embodiment of the invention shown in Figure 3, the elongate body comprises two further lumens: a pull-wire lumen 30 and a delivery conduit extension lumen 32.

The pull-wire lumen 30 houses a portion of a pull-wire 34 that extends from the proximal end of the delivery catheter 12 to be fixedly attached at the distal end of the elongate shaft assembly 14, and/or in the distal tip portion 20 of the delivery catheter 12. As is known in the art, the pull-wire 34 may be manipulated by the user at its proximal end to effect deflection of the distal tip of the delivery catheter 12 to assist the direction and fine positioning the catheter via the well-known Bowden cable method of transmitting movement.

The delivery conduit extension lumen 32 houses a proximal extension of a delivery conduit 36. The delivery conduit 22 provides fluid communication between the mixing assembly 6 and a site of delivery of the one or more fluid components exiting the mixing assembly. The proximal extension of the delivery conduit 36 extends from the proximal end of the delivery catheter 12 to the delivery conduit 22 at or near its distal end. The delivery conduit extension 36 allows for remote manipulation of the end of the delivery conduit 22, for example to deploy or retract the delivery conduit 22 from the proximal end of the delivery catheter 12.

The mixing assembly 6 is disposed co-axially within the elongate shaft assembly 14 of the delivery catheter 12 at or near its distal end. The mixing assembly 6 comprises a mixing assembly body 38 having at least one void, or a mixing or a combining chamber 40. The mixing chamber 40 extends at least part way along the length of the mixing assembly body 38. Suitably, the mixing chamber 40 extends substantially, or entirely, along the length of the mixing assembly body 38. As shown in **Figure 4****,** in an embodiment of the invention the body 38 of the mixing assembly 6 further comprises a pull-wire lumen 42. The pull-wire lumen 42 is isolated from the mixing chamber 40 meaning there is no fluid communication between the pull-wire lumen 42 and the mixing chamber 6. This arrangement prevents unwanted leakage of fluid from the mixing chamber 6 and/or contamination of the pull-wire 34 that may interfere with its correct operation.

The mixing or combining chamber 40 accepts fluid components 2, 4 from the separate fluid component lumens 26, 28 of the elongate body 16. The mixing chamber 40 provides a volume in which the fluid components 2, 4 may combine and/or physically interact. In combining, the fluid components 2, 4 may at least partially mix.

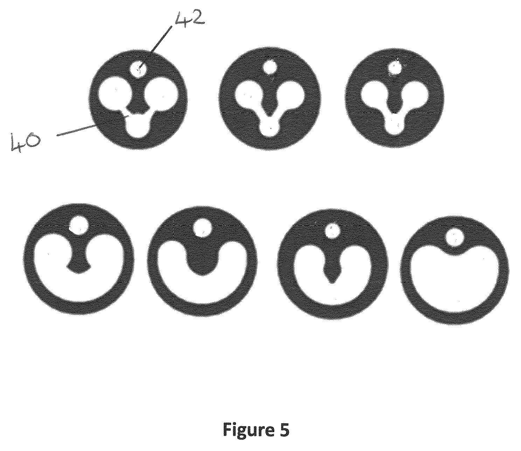

The mixing or combining chamber 40 may have any suitable profile to allow mixing of the fluid components 2, 4. As can be seen from **Figure 5** the mixing chamber profile can have different designs. All these designs have a separated lumen for a pull-wire 42 and a mixing chamber 40 providing a common cavity below where the mixing of the fluid components occurs.

The internal configuration of the mixing or combining chamber 40 may be chosen to encourage mixing of the fluid components 2, 4. In certain embodiments, the internal configuration of the mixing chamber 40 may encourage mixing by generating hydrodynamic turbulence in the fluid components 2, 4. Suitably, the mixing of the fluid components 2, 4 is enhanced or improved compared to that expected by mere co-administration of the fluid components 2, 4. The internal configuration of the mixing chamber 40 may be adapted in any suitable manner to encourage mixing of the fluid components 2, 4. In embodiments, the internal configuration may be adapted by varying the sectional mixing chamber profile as shown in Figure 5. The sectional mixing chamber profile may also be varied along the length of the mixing chamber 40. The overall length of the mixing chamber 40 may also be chosen to optimise mixing of the fluid components 2, 4. It would be within the ambit of the skilled person to choose suitable shape and dimensions of the various components for different fluid components.

Suitably the volume of the mixing chamber 40 is at least 1µl. Typically the volume of the mixing chamber 40 is at least 2µl, 3µl, 4µl or 5 µl. The volume of mixing chamber 40 is suitably at most 50µl. Typically, the volume of the mixing chamber 40 is at most 40µl, 30µl, 20µl, or 15µl. The volume of the mixing chamber can be optimised based on the fluid component properties.

The mixing body 38 may be constructed from stainless steel (e.g. 304 or 316) by machining. Alternatively, it can also be manufactured by extrusion of PEBAX (e.g. 4033), or machined/moulded from polymer or 3D printed.

Any degree of mixing of the fluid components 2, 4 in the mixing chamber 40 is considered encompassed by the invention. In some embodiments of the invention, the fluid components 2, 4 may be chosen so that on mixing they begin to react with each other chemically. Suitably, the fluid components 2, 4 are mixed sufficiently in the mixing chamber 40 to ensure that the desired chemical reaction is initiated in substantially all of the volume of the mixed fluid components 8. The chemical reaction that takes place between the fluid components 2, 4 in the mixing chamber 40 may be a cross-linking and/or a polymerisation reaction. The fluid components 2, 4 may be reactive monomers that when mixed cross-link to form a polymeric product. The polymeric product may be, for example, a biocompatible polymer or gel, such as a bioerodable polymer or gel, or a non-bioerodable polymer or gel. Preferably, the polymer is a bioerodable gel.

The term "biocompatible polymer" refers to polymers or gels which are non-toxic, chemically inert, and substantially non-immunogenic when used internally in a subject and which are substantially insoluble in blood or other bodily fluids. The biocompatible polymer can be either non-biodegradable or biodegradable. Suitably, the biocompatible polymer is also non-inflammatory when employed *in situ.*

Bioerodable biocompatible polymers are known in the art. Examples of suitable bioerodable polymers include, but are not limited to, linear-chain polymers such as polypeptides, polynucleotides, polysaccharides, polylactides, polyglycolides, polycaprolactones, copolymers of polylactic acid and polyglycolic acid, polyanhydrides, polyepsilon caprolactone, polyamides, polyurethanes, polyesteramides, polyorthoesters, polydioxanones, polyacetals, polyketals, polycarbonates, polyorthocarbonates, polydihydropyrans, polyphosphazenes, polyhydroxybutyrates, polyhydroxyvalerates, polyalkylene oxalates, polyalkylene succinates, poly(malic acid), poly(amino acids), polyvinylpyrrolidone, polyethylene glycol, polyhydroxycellulose, polymethyl methacrylate, chitin, chitosan, copolymers of polylactic acid and polyglycolic acid, poly(glycerol sebacate) (PGS), fumaric acid, sebacic acid, and copolymers, terpolymers including one or more of the foregoing. Other bioerodable polymers include, for example, gelatin, collagen, silk, chitosan, alginate, cellulose, poly-nucleic acids, etc.

Suitable non-bioerodable biocompatible polymers include, by way of example, cellulose acetates (including cellulose diacetate), polyethylene, polypropylene, polybutylene, polyethylene terphthalate (PET), polyvinyl chloride, polystyrene, polyamides, nylon, polycarbonates, polysulfides, polysulfones, hydrogels (e.g., acrylics), polyacrylonitrile, polyvinylacetate, cellulose acetate butyrate, nitrocellulose, copolymers of urethane/carbonate, copolymers of styrene/maleic acid, poly(ethylenimine), Poloxamers (e.g. Pluronic such as Poloxamers 407 and 188), Hyaluron, heparin, agarose, Pullulan, and copolymers including one or more of the foregoing, such as ethylene/vinyl alcohol copolymers (EVOH).

In some embodiments, the biocompatible polymer may be a copolymer of polylactic acid and polyglycolic acid, poly(glycerol sebacate) (PGS), poly(ethylenimine), Pluronic (Poloxamers 407, 188), Hyaluron, heparin, agarose, or Pullulan. In some embodiments, the polymer is hyaluronan derivatives in the form of a gel.

The mixed fluid components 8 exit the mixing chamber 40 of the mixing assembly 6 to the delivery conduit 22. The invention is not limited by the means by which the mixed fluid components 8 pass between the mixing chamber 6 and the delivery conduit 22. Generally, the mixed fluid components 8 leave the mixing chamber 40 through an outlet 44 and enter the delivery conduit 22 through an inlet 46. The outlet 44 of the mixing chamber 40 and the inlet 46 of the delivery conduit 22 may be in the form of an aperture 48 in the delivery conduit 22 that accepts mixed fluid components 8 from the mixing chamber 40. Alternatively, the outlet 44 of the mixing chamber 40 and the inlet 46 of the delivery conduit 22 may be separated but maintained in fluid communication via one or more intervening chambers.

The delivery conduit 22 directs the mixed fluid components 8 from the mixing chamber 40 to a delivery terminus 10 and into a delivery site within the body. The delivery conduit 22 may be of any suitable design, including and not limited to a simple opening in, or outlet from, in the mixing chamber 40. Alternatively, it may take the form of a lumen, a tube, a hollow needle, a channel, a pipe, a cylinder, a hose, a duct or a spout in fluid communication with the mixing chamber 40. The choice of delivery conduit 22 may be dependent on the intended delivery site, for example, for a delivery site located in an open vessel, orifice, or receptacle within the body then, for example, an opening, tube or blunt needle may be selected. Alternatively if the intended delivery site is within solid tissue or substrate, the delivery conduit 22 may be required to penetrate the tissue prior to the ejecting the mixed fluid components within, therefore a sharp hollow needle may be selected, for example.

In the embodiment of the invention shown in Figures 2 to 4, the hollow needle 22, which acts as the delivery conduit, extends into the mixing chamber 40. There is provided in the wall of the hollow needle 22 an aperture 48. More than one aperture 48 may be provided the wall of the needle 22 in alternative embodiments. The aperture 48 in the wall of the delivery conduit 22 or hollow needle is positioned so that it may be juxtaposed with the interior of the mixing chamber 40. When configured in this manner, the aperture may act both as an outlet 44 for the mixing chamber 40 and an inlet 46 to the interior lumen of the hollow needle 22 (the delivery conduit). The aperture 48 allows fluid communication between the mixing chamber 40 and an interior lumen of the needle 22.

In use, positive pressure may be applied to the fluids in the mixing chamber 40. In an embodiment, syringes attached to the proximal end of the fluid component lumens may be used to apply pressure to the fluid within. Alternatively, a pump may be employed to apply a positive pressure. The pressure applied is transferred hydraulically through the fluid network within the apparatus to force fluid components 2, 4 from the lumens 26, 28 into the mixing chamber 40 where they will combine and may at least partially mix. The positive pressure urges the mixed fluid components through the mixing chamber 40 and to the delivery conduit 22, leading to flow of the mixed fluid components 8 in a proximal to distal direction, and thereby, dispensing of the mixed fluid components 8 from the distal delivery terminus 10 of the hollow needle 22. The flow ceases in the absence of positive pressure.

Retrograde flow of mixed fluid components 8 in the proximal direction along the delivery conduit 22, in this embodiment a needle, may be prevented by introducing a restriction or obstruction 50 in an interior lumen defined by the hollow needle 22 proximate to, and proximal of, the aperture 48. In an embodiment, such a restriction 50 may be placed at the juncture between the delivery conduit 22 and the delivery conduit extension 33. Alternatively, in embodiments where there is no restriction in the lumen of the delivery conduit 22, but the proximal end of the needle 22 is sealed, retrograde flow of mixed fluid components 8 in the proximal direction along the needle may be minimised or prevented by back-pressure of the gas trapped inside the needle, proximal to the aperture 48.

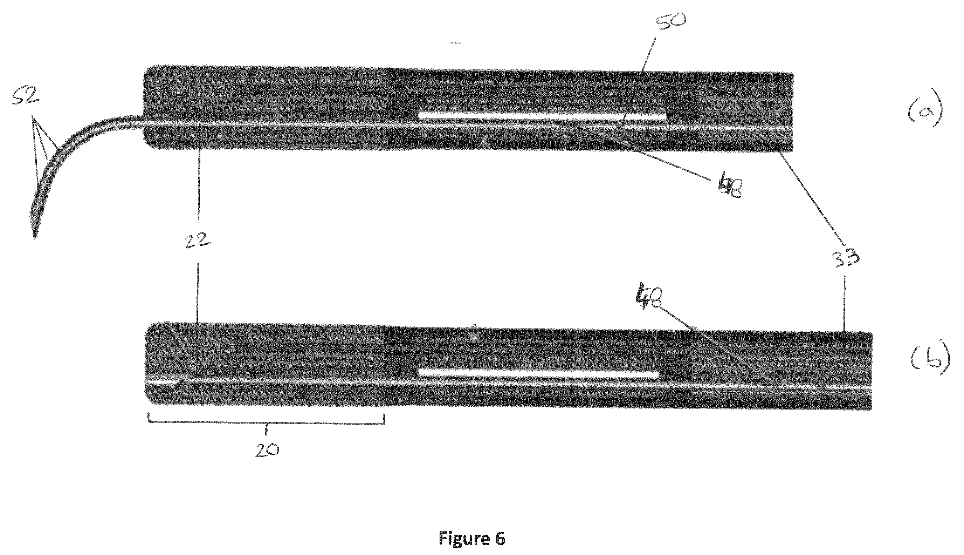

As shown in **Figure 6****,** the delivery conduit 22 may be reciprocated between an at-rest position where the tip of the delivery conduit 22 is entirely retracted within the distal tip portion 20 of the delivery catheter 12, and an extended or deployed position wherein the tip of the delivery conduit 22 extends outside of the delivery catheter 12. In embodiments comprising a delivery conduit extension 33, deployment of the delivery conduit 22 can be effected by the user at the proximal end through longitudinal movements of the delivery conduit extension 33 along the axis of the elongate shaft assembly 14.

In an embodiment, the needle 22 extends out of the distal end face of the elongate shaft assembly 14 of the delivery catheter 12. In this embodiment, the immediate general direction of the needle 22 on deployment from the delivery catheter 12 is along the longitudinal axis (A, Figure 2) of the delivery catheter 12 at its distal end.

It will be appreciated that alternative configurations may also be adopted in which the delivery conduit 22 may be diverted radially at a position proximal to the distal terminus of the elongate shaft assembly 14. Such configurations permit embodiments of the invention in which the delivery conduit 22 radially outwardly from the delivery catheter 12. In this embodiment of the invention the delivery conduit 22 will exit the delivery catheter in an aperture 51 in the side wall of the elongate shaft assembly 14 rather than at its end.

In embodiments where the delivery conduit 22 is a retractable hollow needle or stylet, the delivery conduit 22, may be formed from any suitable material including polyether ether ketone (PEEK), carbon fibre loaded liquid crystalline polymer, tungsten carbide polyimide, stainless steel, gold, platinum, shape memory alloy (including NiTinol) or other suitable surgically compatible metal alloys. Typically, the delivery conduit 22 is formed from a radiopaque material so as to facilitate visualisation during surgical procedures when using X-ray guidance. The delivery conduit 22 may further comprise one or more echogenic surfaces to further facilitate use with ultrasound visualisation (e.g. IVUS) technologies.

As it deploys, the needle 22 may remain straight, or as shown in Figures 2 to 4, it may assume a curved shape deflecting the tip of the needle radially from the longitudinal axis (A, Figure 2) of the delivery catheter 12 at its distal end. To achieve this curvature, the needle 22 may be formed of a shape-memory alloy, such as NiTinol, which is pre-formed in the curved shape, and is able to adopt the required straight orientation when retracted within the lumen of the delivery catheter 12.

In embodiments of the invention where the delivery conduit 12 comprises a hollow needle, the needle may have a plurality of perforations 52 in its side wall near its distal tip. The perforations 52 may increase in number and/or size as the distal end of the needle 22 is approached. Such an arrangement may lead to a beneficial distribution pattern of the ejected material, in terms of both direction and uniformity of flow from the proximal to distal perforations 52. This may lead to further improved retention of therapeutic agent though distributing the ejected material more evenly and widely within the tissue.

In an embodiment of the invention, the delivery conduit 22 is a single 28G needle (outside diameter = 0.356mm) to minimise trauma to the tissues of the patient into which the needle penetrates.

In the embodiment shown in Figures 2 to 4 the mixing chamber is sealed at its proximal and distal ends by first and second end walls: a first proximal end wall 54, and a second distal end wall 56. In an embodiment, the proximal end wall of the mixing assembly body 38 abuts the distal end of, and separates the mixing assembly 6from, the elongate body 16. The distal end wall 56 abuts the distal end of the mixing assembly 18, and separates it from the distal tip portion 20 of the delivery catheter 12.

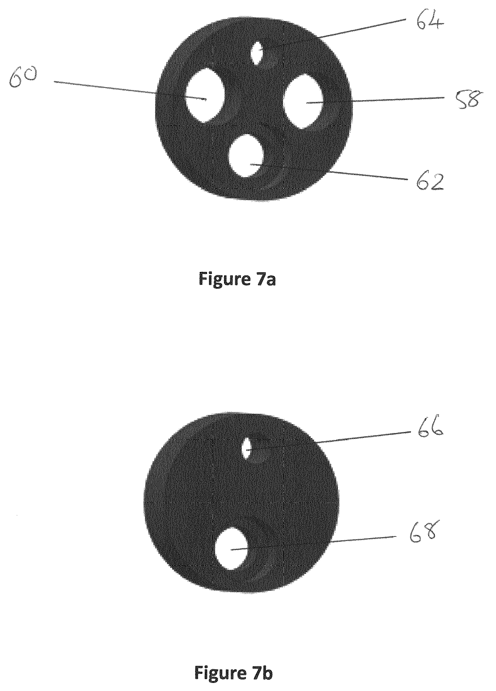

As shown in **Figure 7a****,** in an embodiment of the invention, the proximal end wall 54 of the mixing assembly 6has four apertures or openings. A first and second fluid component opening 58, 60 are sized and positioned to be in sealed aligned registry with the first and second component lumens 26, 28 in the elongate body 16 and the mixing chamber 40 in the mixing assembly 18. Fluid communication between the first and second component lumens and the mixing chamber 40 is thereby permitted. A delivery conduit opening 62 in the proximal end wall is sized and positioned to be in aligned registry with the distal end of the delivery conduit extension lumen 32 in the elongate body 16. A pull wire opening 64 in the proximal end wall 54 is sized and positioned to be in aligned registry with the distal end of the pull-wire extension lumen 30 in the elongate body 16. As shown in **Figure 7b****,** the distal end wall has apertures or openings 66, 68 that are sized and positioned to be in aligned registry only with the lumens in the mixing assembly body 38 that house the pull-wire 34 and the delivery conduit 22. This arrangement permits the unhindered passage of the pull-wire 34 and the delivery conduit 22 from the elongate body 16 through the mixing assembly body 38 to the distal tip portion 20 of the delivery catheter 12.

To prevent unwanted leakage from the mixing chamber, in an embodiment of the invention, O-ring seals 70, 72 are positioned within the openings 62, 68 in the proximal end wall 54 and the distal end wall 56 that surround the delivery conduit 22 to form a fluid tight seal to the mixing chamber that may be maintained during longitudinal movement of the delivery conduit 22 when it moves between its retracted and its extended position.

In embodiments of the invention, there is provided a distal tip portion 20 that is located distal of the mixing assembly 18, at the end of the elongate shaft assembly 14. The distal tip portion 20 may comprise a radiopaque material or coating so as to facilitate visualisation during surgical procedures when using X-rays. The distal tip portion 20 may further comprise one or more echogenic surfaces to further facilitate use with ultrasound visualisation (e.g. IVUS) technologies.

In an embodiment, the mixed fluid components 8 exit the delivery conduit 22 as a free-flowing fluid, as opposed to a semi-solid gel, or a solid mass. This facilitates the expulsion of the mixed components 8 from the catheter 12 and prevents undesirable use of high pressure to expel the components and/or blockage of the device. For a typical embodiment of the invention it is anticipated that the maximum positive pressure necessary to advance the mixed fluid components 8 from the mixing chamber 40 should be at or below that which can be achieved by the operation of a typical luer lock syringe operated by a user at the proximal end of the device. For certain embodiments, additional pressure could be applied by a pump or other suitable fluid pressure generating equipment.

The applied hydraulic pressure required to push the mixed fluid components 8 from the mixing chamber 40, and hence to dispense the mixed fluid components 8 from the apparatus, is dependent on the viscosity of the mixture. The viscosity of the mixture will depend on a number of factors including the viscosity of the unmixed fluid components 2, 4, and in particular, in embodiments where the fluid components react to form a polymer, the extent of polymerisation of the mixture. The extent of polymerisation may change over time with the viscosity of the mixture expected to generally increase with progression of the reaction by which the extent of cross-linking increases.

In embodiments where the viscosity of the mixed fluid components increases with time, the time taken between the fluid components becoming mixed to being expelled from the apparatus, referred to hereinafter as the "residence time", is a key factor. The residence time may be varied by adjusting the overall flow rate of fluid components through the apparatus. Typically, the flow rate is determined by the flow rate through the most restricted point. In the embodiment shown in Figures 2 to 4, the most restricted point is the aperture 48 in the wall of the delivery conduit 22. In an embodiment of the invention, the shape and size of the aperture 48 to provide a residence time that permits the dispensing of mixed fluid components 8 from the apparatus while the viscosity is appropriate for the positive pressure required for dispensing to be in the optimal operating range. It is envisaged that the diameter of the fluid component lumens 26, 28 and/or the delivery conduit 22 may also be used to vary the flow rate and hence the residence time.

The invention is further exemplified by the following non-limiting clauses:
1. An apparatus, suitable for use as a delivery catheter, the apparatus comprising:
   (a) an elongate shaft that comprises a proximal end and a distal end, wherein the elongate shaft defines at least one lumen; and
   (b) at least one combining chamber that is contained within the lumen proximate to the distal end of the elongate shaft, the combining chamber having at least a first and a second inlet to allow a first and a second fluid component to enter the combining chamber; and at least one outlet to allow the combined first and second fluid components to exit the combining chamber.
2. The apparatus of clause 1, wherein the apparatus further comprises at least a first and a second lumen that extend to the proximal end of the elongate shaft from the combining chamber, wherein the first and second inlets are in respective fluid communication with the first and second lumens.
3. The apparatus of clause 2, wherein the first and second lumens are connected to a first and a second component reservoir located at the proximal end.
4. The apparatus of clause 3, wherein the first and second component reservoirs comprise a first and a second syringe.
5. The apparatus of clause 4, wherein the means of attachment of the first and the second syringe to the respective first and second lumen is via a luer lock coupling.
6. The apparatus of any one of clauses 1 to 5, wherein in use the first and second fluid components are combined within the combining chamber.
7. The apparatus of clause 6, wherein the first and second fluid components are at least partially mixed within the combining chamber.
8. The apparatus of clause 7, wherein the first and second fluid components form a biocompatible polymer when combined.
9. The apparatus of clause 8, wherein the first and second fluid components are mixed within the combining chamber to the extent that a polymerisation reaction is initiated in substantially all of the volume of the mixed fluid components.
10. The apparatus of clause 7 or clause 8, wherein the biocompatible polymer is a bioerodable gel.
11. The apparatus of any one of clauses 1 to 10, wherein the combined first and second fluid components are dispensed from the apparatus as a fluid.
12. The apparatus of clause 11, wherein the flow rate of the first and second fluid components through the apparatus is controlled such that the combined first and second fluid components are dispensed as a fluid.
13. The apparatus of clause 12, wherein the means of control of the flow rate is selected from the group consisting of: rate of introduction of the first and second fluid components into the combining chamber; size of the at least two inlets to the combining chamber; size of the at least one outlet from the combining chamber; viscosity of the first and second fluid components; viscosity of mixed first and second fluid components; or a combination thereof.
14. The apparatus of any one of clauses 1 to 13, wherein one or more of the first and/or second fluid component contains a therapeutic agent.
15. The apparatus of clause 14, wherein the therapeutic agent is contained in the first fluid component.
16. The apparatus of clause 14, wherein the therapeutic agent is contained in more than one fluid component.
17. The apparatus of clause 14, wherein the combined first and second fluid components that are dispensed from the apparatus contain a therapeutic agent.
18. The apparatus of any one of clauses 14 to 17, wherein the therapeutic agent comprises cells.
19. The apparatus of clause 18, wherein the cells are stem cells.
20. The apparatus of any one of clauses 1 to 19, wherein the combining chamber defines a dead volume of at least 1µl, optionally at least 5µl.
21. The apparatus of any one of clauses 1 to 20, wherein the combining chamber defines a dead volume of at most 50µl, optionally at least 15µl.
22. The apparatus of any one of clauses 1 to 19, wherein the combining chamber defines a dead volume in the range of 1µl to 50µl, optionally, in the range of 5µl to 15µl.
23. The apparatus of any one of clauses 1 to 22, wherein the apparatus further comprises a delivery conduit wherein the delivery conduit receives combined first and second fluid components from the combining chamber via the at least one outlet and directs the combined first and second fluid components to a delivery site.
24. The apparatus of clause 23, wherein the delivery site is within a body tissue, joints, a body cavity or vessel.
25. The apparatus of clause 24, wherein the delivery site is within the myocardium.
26. The apparatus of any one of clauses 23 to 25, wherein the delivery conduit moves between a retracted position wherein the distal end of the delivery conduit is located within the central lumen of the apparatus, and an extended position wherein the distal end of the delivery conduit extends outside of the central lumen of the apparatus.
27. The apparatus of any one of clauses 23 to 26, wherein the delivery conduit is connected to the combining chamber.
28. The apparatus of clause 27, wherein the delivery conduit extends into the combining chamber.
29. The apparatus of clause 28, wherein the delivery conduit extends from the distal end of the apparatus through the combining chamber to the proximal end of the apparatus.
30. The apparatus of any one of clauses 23 to 29, wherein the delivery conduit receives combined first and second fluid components via at least one aperture in the side wall of the delivery conduit.
31. The apparatus of clause 30, wherein the at least one aperture permits fluid communication between the combining chamber and the delivery conduit when the delivery conduit is in the extended position.
32. The apparatus of clause 30 or clause 31, wherein the at least one aperture does not permit fluid communication between the combining chamber and the delivery conduit when the delivery conduit is in the retracted position.
33. The apparatus of any one of clauses 23 to 32, wherein the delivery conduit comprises a restriction proximal to the aperture in the delivery conduit thereby promoting unidirectional flow of the mixed first and second fluid components through the delivery conduit in a proximal to distal direction.
34. The apparatus of any one of clauses 23 to 33, wherein the delivery conduit is a hollow needle.
35. The apparatus of clause 34, wherein the hollow needle is formed of shape-memory metal.
36. An elongate shaft assembly for use as a delivery catheter, the elongate shaft assembly comprising:
   (a) an elongate body that comprises a proximal end and a distal end, wherein the elongate body comprises at least a first lumen and a second lumen;
   (b) at least one mixing chamber that is located at the distal end of the elongate body; and
   (c) a delivery conduit connected to the mixing chamber;
   wherein the mixing assembly is located in the vicinity of the distal end of the elongate shaft assembly; and wherein the mixing chamber is arranged to be in fluid communication with each of the first and second lumens.
37. The elongate shaft assembly of clause 36, wherein the first and second lumens extend through the elongate body to the proximal end of the elongate shaft assembly.
38. The elongate shaft assembly of clause 36, wherein the first lumen and the second lumen are attached to a respective first fluid component reservoir and a second fluid component reservoir located at the proximal end of the elongate shaft assembly.
39. The elongate shaft assembly of clause 38, wherein the first and second fluid component reservoirs comprise a respective first syringe and a second syringe.
40. The elongate shaft assembly of clause 39, wherein the means of attachment of the first and the second syringe to the respective first and second lumen is via a luer lock coupling.
41. The elongate shaft assembly of any one of clauses 36 to 40, wherein in use a first fluid component and a second fluid component are at least partially mixed within the mixing chamber to provide mixed first and second fluid components.
42. The elongate shaft assembly of clause 41, wherein the first and second fluid components form a biocompatible polymer when mixed.
43. The elongate shaft assembly of clause 42 wherein the first and second fluid components are mixed within the mixing chamber to the extent that a polymerisation reaction is initiated in substantially all of the volume of the mixed fluid components.
44. The elongate shaft assembly of clause 42 or clause 43, wherein the biocompatible polymer is a bioerodable gel.
45. The elongate shaft assembly of any one of clauses 41 to 44, wherein the mixed first and second fluid components are dispensed from the delivery conduit as a fluid.
46. The elongate shaft assembly of any one of clauses 41 to 45, wherein the flow rate of the first and second fluid components through the apparatus is controlled such that the mixed first and second fluid components are dispensed as a fluid.
47. The elongate shaft assembly of clause 46, wherein the means of control of the flow rate is selected from the group consisting of: rate of introduction of the first and second fluid components into the at least one mixing chamber; size of the inlets to the at least one mixing chamber; size of the outlet from the at least one mixing chamber; viscosity of first and second fluid components; viscosity of the mixed first and second fluid components; or a combination thereof.
48. The elongate shaft assembly of any one of clauses 41 to 47, wherein one or more of the first and second fluid components contains a therapeutic agent.
49. The apparatus of clause 48, wherein the therapeutic agent is contained in the first fluid component.
50. The apparatus of clause 48, wherein the therapeutic agent is contained in both the first fluid component and the second fluid component.
51. The elongate shaft assembly of any one of clauses 41 to 50, wherein the combined first and second fluid components that are dispensed from the apparatus contain a therapeutic agent.
52. The elongate shaft assembly of any one of clauses 48 to 50, wherein the therapeutic agent is cells.
53. The elongate shaft assembly of any one of clauses 48 to 50, wherein the cells are stem cells.
54. The elongate shaft assembly of any one of clauses 36 to 53, wherein the mixing chamber defines a dead volume of at least 1µl, optionally at least 5µl.
55. The elongate shaft assembly of any one of clauses 36 to 54, wherein the mixing chamber defines a dead volume of at most 50µl, optionally at least 15µl.
56. The elongate shaft assembly of any one of clauses 36 to 53, wherein the mixing chamber defines a dead volume in the range of 1µl to 50µl, optionally, in the range of 5µl to 15µl.
57. The elongate shaft assembly of any one of clauses 36 to 56, wherein the delivery conduit is an opening in the mixing chamber.
58. The elongate shaft assembly of clause any one of clauses 36 to 57, wherein the delivery conduit is an elongate hollow member selected from the group consisting of: a lumen, a tube, a hollow needle, a channel, a pipe, a cylinder, a hose, a duct or a spout.
59. The elongate shaft assembly of any one of clauses 36 to 58, wherein the delivery conduit moves between a retracted position wherein the distal end of the delivery conduit is located within the elongate shaft assembly of the apparatus, and an extended position wherein the distal end of the delivery conduit extends outside of the elongate shaft assembly of the apparatus.
60. The elongate shaft assembly of any one of clauses 36 to 59, wherein the delivery conduit is connected to the mixing chamber.
61. The elongate shaft assembly of clause 60, wherein the delivery conduit extends into the mixing chamber.
62. The elongate shaft assembly of any one of clauses 36 to 61, wherein the delivery conduit extends from the distal end of the apparatus through the combining chamber to the proximal end of the apparatus.
63. The elongate shaft assembly of any one of clauses 36 to 62, wherein fluid communication between the mixing chamber and the delivery conduit is via at least one aperture in the side wall of the delivery conduit.
64. The apparatus of clause 63, wherein the at least one aperture permits fluid communication between the mixing chamber and the delivery conduit when the delivery conduit is in the extended position.
65. The elongate shaft assembly of any one of clauses 36 to 64, wherein the at least one aperture does not permit fluid communication between the mixing chamber and the delivery conduit when the delivery conduit is in the retracted position.
66. The elongate shaft assembly of any one of clauses 36 to 65, wherein the delivery conduit comprises a restriction proximal to the aperture in the delivery conduit thereby promoting unidirectional flow of the mixed elongate shaft assembly fluid components through the delivery conduit in a proximal to distal direction.
67. The elongate shaft assembly of any one of clauses 36 to 66, wherein the delivery conduit is a hollow needle.
68. The apparatus of clause 67, wherein the hollow needle is formed of shape-memory metal.
69. The apparatus of any one of clauses 1 to 35 or the elongate shaft assembly of any one of clauses 36 to 68, further comprising a pull-wire that is slidably located within a pull-wire lumen within the elongate shaft.
70. The apparatus or the elongate shaft assembly of clause 69, wherein the pull-wire extends from the proximal end of the apparatus to be fixedly attached at the distal end of the apparatus.
71. The apparatus or the elongate shaft assembly of clause 69 or clause 70, wherein the pull-wire acts to deflect the distal tip of the apparatus.
72. A percutaneous catheter comprising an apparatus or elongate shaft assembly as clauseed in any one of clauses 1 to 71.
73. The percutaneous catheter of clause 72, wherein the percutaneous catheter is an injection catheter.
74. A method for directing therapy within the body of a subject comprising using an apparatus or elongate shaft assembly as clauseed in any one of clauses 1 to 73.
75. The method of clause 74, wherein the therapy comprises delivery of a pharmaceutical composition.
76. The method of clause 74, wherein the therapy comprises delivery of a cellular preparation.

It should be understood that the different embodiments of the invention described herein can be combined where appropriate and that features of the embodiments of the invention can be used interchangeably with other embodiments where appropriate.

Although particular embodiments of the invention have been disclosed herein in detail, this has been done by way of example and for the purposes of illustration only. The aforementioned embodiments are not intended to be limiting with respect to the scope of the appended claims, which follow. It is contemplated by the inventors that various substitutions, alterations, and modifications may be made to the invention without departing from the scope of the invention as defined by the claims.

## Claims

1. An apparatus, suitable for use as a percutaneous delivery catheter, the apparatus comprising:
(a) an elongate shaft that comprises a proximal end and a distal end, wherein the elongate shaft defines at least one lumen; and
(b) at least one combining chamber that is contained within the lumen proximate to the distal end of the elongate shaft, the combining chamber having at least a first and a second inlet to allow a first and a second fluid component to enter the combining chamber; and at least one outlet to allow the combined first and second fluid components to exit the combining chamber.

2. The apparatus of claim 1, wherein the apparatus further comprises at least a first and a second lumen that extend to the proximal end of the elongate shaft from the combining chamber, wherein the first and second inlets are in respective fluid communication with the first and second lumens.

3. The apparatus of claim 2, wherein the first and second lumens are connected to a first and a second component reservoir located at the proximal end.

4. The apparatus of any one of claims 1 to 3, wherein in use the first and second fluid components are combined within the combining chamber.

5. The apparatus of claim 4, wherein the first and second fluid components form a biocompatible polymer when combined, and wherein the first and second fluid components are mixed within the combining chamber to the extent that a polymerisation reaction is initiated in substantially all of the volume of the mixed fluid components.

6. The apparatus of any one of claims 1 to 5, wherein the combined first and second fluid components are dispensed from the apparatus as a fluid, and wherein the fluid comprises a bioerodable gel.

7. The apparatus of any one of claims 1 to 6, wherein one or more of the first and/or second fluid component contains a therapeutic agent.

8. The apparatus of claim 7, wherein the therapeutic agent comprises cells, and wherein the cells are optionally stem cells.

9. The apparatus of any one of claims 1 to 8, wherein the apparatus further comprises a delivery conduit wherein the delivery conduit receives combined first and second fluid components from the combining chamber via the at least one outlet and directs the combined first and second fluid components to a delivery site.

10. The apparatus of claim 9, wherein the delivery site is within a body tissue, joints, a body cavity or vessel.

11. The apparatus of claim 10, wherein the delivery site is within the myocardium.

12. The apparatus of any one of claims 9 to 11, wherein the delivery conduit moves between a retracted position wherein the distal end of the delivery conduit is located within the central lumen of the apparatus, and an extended position wherein the distal end of the delivery conduit extends outside of the central lumen of the apparatus.

13. The apparatus of any one of claims 9 to 12, wherein the delivery conduit receives combined first and second fluid components via at least one aperture in the side wall of the delivery conduit.

14. The apparatus of claim 13, wherein the at least one aperture permits fluid communication between the combining chamber and the delivery conduit when the delivery conduit is in the extended position.

15. The apparatus of any one of claims 9 to 14, wherein the delivery conduit is a hollow needle.
